(19) Europäisches Patentamt
European Patent Office
Office européen des brevets

(11) Veröffentlichungsnummer : **0 408 078 B1**

(12) # EUROPÄISCHE PATENTSCHRIFT

(45) Veröffentlichungstag der Patentschrift : **15.11.95 Patentblatt 95/46**

(51) Int. Cl.$^6$ : **G01N 33/547,** // G01N33/543

(21) Anmeldenummer : **90113486.6**

(22) Anmeldetag : **13.07.90**

(54) **Verfahren zur Herstellung einer mit einer immunologisch aktiven Substanz beschichteten Festphasenmatrix.**

(30) Priorität : **14.07.89 DE 3923342**

(43) Veröffentlichungstag der Anmeldung : **16.01.91 Patentblatt 91/03**

(45) Bekanntmachung des Hinweises auf die Patenterteilung : **15.11.95 Patentblatt 95/46**

(84) Benannte Vertragsstaaten : **AT BE CH DE DK ES FR GB GR IT LI LU NL SE**

(56) Entgegenhaltungen :
**EP-A- 0 175 973**
**EP-A- 0 319 957**
**EP-A- 0 410 323**
**WO-A-89/01160**
**DE-A- 2 533 701**
**US-A- 4 689 310**

(73) Patentinhaber : **BOEHRINGER MANNHEIM GMBH**
**D-68298 Mannheim (DE)**

(72) Erfinder : **Maurer, Eberhard, Dr. rer. nat.**
**Blumenstrasse 10**
**D-8120 Weilheim (DE)**
Erfinder : **Deeg, Rolf, Dr. rer. nat.**
**Hirtenstrasse 7**
**D-8139 Bernried (DE)**

(74) Vertreter : **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

**EP 0 408 078 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft ein Verfahren zur Herstellung einer mit einer immunologisch aktiven Substanz beschichteten Festphasenmatrix.

In der Diagnostik werden für viele Bestimmungen, die nach dem Prinzip des heterogenen Immunoassays ablaufen, Festphasen verwendet, an denen eine Substanz gebunden ist, die die zu bestimmende Substanz oder einen Komplex aus zu bestimmender Substanz und markierter Substanz binden kann. Die Bindung dieses spezifisch bindefähigen Materials an eine Festphase ist dabei ein großes Problem. Abhängig von der Art des Trägermaterials kann die Bindung adsorptiv oder kovalent erfolgen. Eine kovalente Bindung erfordert in der Regel Trägermaterialien, die entsprechende funktionelle Gruppen haben und immunologisch aktive Substanzen, die ebenfalls entsprechende funktionelle Gruppen aufweisen. Diese Verfahren sind technisch sehr aufwendig und beeinflussen häufig die Aktivität der immunologisch aktiven Substanzen. Die adsorptive Bindung, die in der Regel günstiger ist zur Aktivitätserhaltung, hat den Nachteil, daß an Kunststoffoberflächen nur eine unzureichende Haftung erreicht werden kann. Deshalb kommt es im Rahmen der Inkubation, die für die Durchführung der Bestimmungsverfahren notwendig ist und aufgrund von Temperatur- und Wascheinflüssen zu Desorptionseffekten und Verdrängung durch Serumbestandteile oder Detergentien. Ein großer Nachteil der häufig angewandten, rein adsorptiven Bindung von Proteinen an Kunststoffoberflächen ist die unzureichende Haftung, die viele Probleme verursacht. So können von der Wand abgelöste Wandantikörper in der Testlösung mit dem Analyten reagieren und diesen dadurch abfangen, was falsche Wiederfindungswerte ergibt. Weiterhin können stark bindende Serumbestandteile Wandantikörper verdrängen, was ebenfalls zu falschen Wiederfindungswerten führt. Eine Testführung bei erhöhter Temperatur, die wegen kürzerer Reaktionszeiten angestrebt wird, ist wegen stark zunehmender Desorption oft nicht möglich wegen Signalabfall und temperaturabhängiger Wiederfindungen. Intensive Waschschritte, die zur Vermeidung der Verschleppung und zur Entfernung unspezifisch gebundener Serumbestandteile durchgeführt werden sollten, führen zu mehr oder weniger starker Ablösung von Wandantikörpern, was die Wiederfindung verfälscht und die Präzision vermindert. Auch die zur Vermeidung unspezifischer Bindungen häufig eingesetzten Detergentien beeinträchtigen die adsorptive Bindung und führen damit zu starker Wandablösung. Aufgrund von Schwankungen der adsorptiven Bindungseigenschaften des Trägermaterials, die chargenabhängig sind, werden unterschiedliche Ablöseraten erhalten, was wiederum die Präzision mindert und falsche Wiederfindungen ergibt. Durch all diese Probleme werden Präzision und Empfindlichkeit von immunologischen Bestimmungsverfahren beeinträchtigt.

Um die Bindung der immunologisch aktiven Substanz an der Festphase zu verbessern, wurde in US-PS 4,689,310 vorgeschlagen, entweder an das Trägermaterial oder an den zu bindenden Liganden kovalent eine Verbindung zu knüpfen, die eine fotoaktivierbare Gruppe aufweist. Wenn man dann Trägermaterial und Ligand, von denen einer entsprechend derivatisiert ist, in Kontakt bringt und bestrahlt, so kann eine Bindung zwischen beiden erfolgen. Dieses Verfahren ist jedoch aufwendig und liefert auch keine befriedigenden Ergebnisse.

EP-A-0 319 957 offenbart ein Verfahren zur Fixierung einer Substanz, z.B. einer immunologisch aktiven Substanz, an die Oberfläche eines Trägermaterials. Diese Fixierung erfolgt über eine Verbindung, die eine photoaktivierbare Gruppe enthält. Hierzu kann beispielsweise ein Komplex aus der photoaktivierbaren Verbindung und der zu immobilisierenden Substanz hergestellt und anschließend durch Photoaktivierung an den Träger gebunden werden. Hierbei werden die Moleküle der immunologisch aktiven Substanz ohne jegliche Quervernetzung einzeln an den Träger gebunden.

WO-A-89/01160 offenbart die Vernetzung von immunologisch aktiven Substanzen über Verbindungen mit zwei photoaktivierbaren Gruppen an einen Polymerträger. Diese Vernetzung erfolgt derart, daß die photoreaktive Verbindung zunächst in einer Form, worin eine der beiden photoaktivierbaren Gruppen mit einer Schutzgruppe blockiert ist, mit einer zu immobilisierenden Substanz unter Bildung eines kovalent verknüpften Komplexes umgesetzt wird und dieser Komplex anschließend nach Entfernung der Schutzgruppe kovalent mit einem polymeren Träger in Kontakt gebracht und mit diesem kovalent verknüpft wird.

Es war nun Aufgabe der vorliegenden Erfindung, ein Verfahren zur Verfügung zu stellen, das einfach durchgeführt werden kann und zu einer sehr dauerhaften Bindung einer immunologisch aktiven Substanz am Trägermaterial führt.

Diese Aufgabe wird gelöst durch ein Verfahren zur Herstellung einer mit einer immunologisch aktiven Substanz beschichteten Festphasenmatrix, das dadurch gekennzeichnet ist, daß man ein Trägermaterial mit einer Mischung aus

(a) immunologisch aktiver Substanz,

(b) einer Verbindung, die mindestens zwei fotoaktivierbare funktionelle Gruppen und mindestens einen mit dem Trägermaterial und der immunologisch aktiven Substanz adsorptiv wechselwirkenden hydrophoben Anteil enthält, absorptiv beschichtet und anschließend zur Vernetzung bestrahlt.

Erfindungsgemäß können Festphasenmatrizes in einfacher Weise mit immunologisch bindefähigen Substanzen beschichtet werden, die über einen längeren Zeitraum und auch unter Temperatur- und Detergenseinfluß stabil bleiben.

Das zu beschichtende Trägermaterial wird mit einer Beladelösung in Kontakt gebracht, die die immunologisch aktive Substanz und die fotoaktivierbare Gruppen enthaltende Verbindung enthält. Nach einer ausreichend langen Kontaktzeit, bei der Licht einer Wellenlänge, die zu einer Aktivierung der fotoaktivierbaren Gruppen führt, ausgeschlossen werden muß, bildet sich aufgrund des hydrophoben Anteils durch Wechselwirkung zwischen Protein, der bifunktionellen Verbindung und dem Trägermaterial eine zunächst adsorptiv gebundene Oberflächenschicht aus. Nach Beendigung dieser ersten Inkubation wird mit Licht geeigneter Wellenlänge und Intensität bestrahlt. Hierdurch werden hochreaktive Gruppierungen wie z.B. Carbene und/oder Nitrene an Komponente (b) erzeugt, die in einer schnellen und unspezifischen Reaktion sowohl mit dem Protein als auch mit dem Trägermaterial unter Ausbildung kovalenter Bindungen reagieren. Die zunächst adsorptiv vorgebildete Oberflächenschicht wird auf diese Weise kovalent fixiert durch Protein-Protein- und Protein-Wand-Bindungen. Durch die Kombination dieser Maßnahmen wird die Effizienz des Beschichtungsprozesses und die Vernetzungswahrscheinlichkeit bedeutend verbessert. Weiterhin wird durch die adsorptive Anreicherung von Protein an der Oberfläche die eingesetzte immunologisch aktive Substanz optimal ausgenutzt und eine hohe Beladungsdichte erzielt.

Die erfindungsgemäß hergestellten Festphasenmatrizes werden verwendet für die Durchführung von heterogenen Immunoassays. Dazu wird an der Festphase eine immunologisch aktive Substanz immobilisiert. Unter immunologisch aktiver Substanz werden hier Verbindungen verstanden, die mit anderen Substanzen eine spezifische Bindung eingehen können. Als Beispiele können Antikörper, Bindeproteine, Lectine, Biotin, Avidin bzw. Streptavidin, Hormone und andere biologisch aktive Substanzen genannt werden. Für die Verwendung bei Immunoassays kommen insbesondere immunologisch aktive Substanzen in betracht, die mit der nachzuweisenden Substanz oder einem Komplex aus nachzuweisender Substanz und daran gebundenem markiertem Konjugat bindefähig sind. Bevorzugt werden daher Antikörper und Bindeproteine sowie Streptavidin bzw. Avidin oder Biotin-Konjugate eingesetzt. Ebenso geeignet sind biologisch aktive Moleküle wie T4, T3 auch als Polyhapten, Prolactin, LH oder TSH sowie gegen diese gerichtete Antikörper.

Als Trägermaterial können die an sich üblichen Materialien verwendet werden. Geeignet sind alle Stoffe, die mit einer fotoaktivierbaren Gruppe unter Ausbildung einer kovalenten Bindung reagieren, wie beispielsweise Polysaccharide, Glas, Keramik, Cellulose und Kunststoffe. Durch den Einsatz der fotoaktivierbaren Verbindung können sowohl Kunststoffe, die bereits funktionelle Gruppen aufweisen, als auch bevorzugt inerte Materialien wie z.B. Polyethylen, verwendet werden. Insbesondere sind alle Arten von Kunststoffen, wie z.B. Polyamide, Polyacrylamide, Polyester, Polyethylen, Polypropylen, Silicone, Polycarbonate geeignet. Vorteilhaft sind Kunststoffe, welche die Eigenschaft besitzen, möglichst viel Reaktionspartner adsorptiv zu binden, wie z.B. Polyvinylchlorid, Polymethylmethacrylat (Plexiglas). Für das erfindungsgemäße Verfahren werden besonders bevorzugt Polystyrol und Copolymere des Polystyrols (z.B. mit Acrylnitril → Luran) verwendet. Beim Einsatz von Polystyrol ist es zweckmäßig, das Trägermaterial vor der Beschichtung durch γ-Bestrahlung zu sterilisieren. Das Trägermaterial liegt üblicherweise in Form von Kugeln, Mikrotiterplatten, Röhrchen, Platten, Folien, Geweben/Vliesen, Microgelen/Latex vor.

Die Bindung erfolgt über eine Verbindung, die mindestens zwei fotoaktivierbare funktionelle Gruppen und einen hydrophoben Anteil besitzt und außerdem bevorzugt eine ausreichende Löslichkeit in Wasser bzw. Pufferlösung besitzt. Der hydrophobe Anteil bewirkt dabei die adsorptive Bindung dieser Komponente einerseits an das Trägermaterial und andererseits an die immunologisch aktive Substanz. Wird dann Licht mit geeigneter Wellenlänge und Intensität eingestrahlt, reagieren die entstehenden hochreaktiven Zwischenprodukte (wie z.B. Nitrene, Carbene, Radikale) sowohl mit den Polymer-, als auch mit den Protein-Molekülen. Auf diese Weise entsteht eine intermolekulare Vernetzung des Proteins mit dem Kunststoff. Funktionelle Gruppen, die durch Einwirkung von Licht bestimmter Wellenlänge hochreaktive Zwischenprodukte bilden, sind bekannt. Häufig verwendet werden Azid, Diazo- oder Ketenreste. Die Auswahl der fotoaktivierbaren Gruppierung richtet sich auch nach der Wellenlänge, die zu ihrer Aktivierung erforderlich ist. Da kurzwellige Strahlung eine fotooxidative Zerstörung der Proteine bewirken kann und andererseits die Verwendung von besonders langwelliger Strahlung ungünstig sind, da dann eine zu große Empfindlichkeit gegenüber Tageslicht besteht, sollten die fotoaktivierbaren Gruppen so ausgesucht werden, daß ihre Anregung in einem Bereich von 250 bis 500 nm, besonders bevorzugt 320 bis 400 nm erfolgt. Die erfindungsgemäß verwendete Komponente (b) kann nun zwei oder mehr fotoaktivierbare Gruppen enthalten, die gleich oder verschieden sein können. Bevorzugt wird als Komponente (b) eine homo-bifunktionelle Verbindung verwendet. Der die fotoaktivierbaren Gruppen verbindende Molekülteil ist zumindest teilweise hydrophob. Der hydrophobe Anteil ist dabei bevorzugt so strukturiert, daß das Reagenz sowohl mit der Festphasenoberfläche als auch mit der zu fixierenden Substanz eine möglichst starke hydrophobe Wechselwirkung zeigt. Dazu ist bevorzugt der hydrophobe Anteil ähnlich aufgebaut wie Trägermaterial bzw. immunologisch aktive Substanz. Beispielsweise kann bei der Fixierung von Proteinen

auf Polystyrol- oder Luran-Röhrchen der hydrophobe Anteil kondensierte oder mehrkernige aromatische Ringsysteme enthalten. Bevorzugt wird als Komponente (b) eine Verbindung der folgenden allgemeinen Formel:

V-Hy-W

verwendet, worin V und W jeweils eine fotoaktivierbare funktionelle Gruppe und Hy eine hydrophobe Gruppe bedeuten. Bevorzugt werden dabei als hydrophobe Gruppen der Diphenyl-, Naphthyl- oder Anthracyl-Rest in unsubstituierter oder auch substituierter Form verwendet. In einer besonders bevorzugten Ausführungsform der Erfindung wird als Komponente (b) eine Verbindung der folgenden Formel:

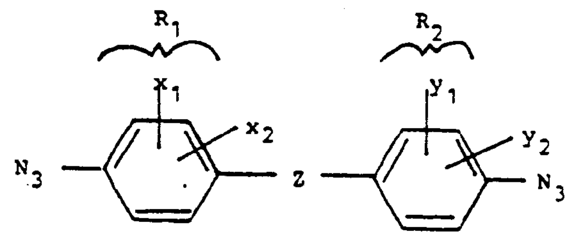

verwendet, worin $R_1$ und $R_2$ die hydrophoben Anteile bezeichnen und $x_1$, $x_2$ und $y_1$, $y_2$ jeweils unabhängig voneinander H, $NO_2$ oder Halogen und Z eine Spacer-Gruppe bedeuten. Die Spacer-Gruppe besteht bevorzugt aus den Elementen C, H, 0, N und/oder S und hat bevorzugt eine Kettenlänge von 1 bis 20 Atomen. Besonders bevorzugt steht Z für die Dithiogruppe.

Immunologisch aktive Substanz und fotoaktivierbare Gruppen enthaltende Verbindung werden in einem solchen Verhältnis eingesetzt, daß die Vernetzung und Bindung der immunologisch aktiven Substanz gesichert ist. Als geeignet hat sich hierbei ein molarer Überschuß der fotoaktivierbaren Verbindung von Faktor 1 bis $10^6$, bevorzugt 3 bis $3x10^5$, besonders bevorzugt von 100 bis 10.000 erwiesen.

Nach der Beschichtung erfolgt eine Bestrahlung, um die erwünschte Vernetzung zu bewirken. Die Bestrahlung erfolgt, abhängig von der verwendeten fotoaktivierbaren Gruppe, bei einer Wellenlänge, die hochreaktive Zwischenprodukte erzeugt, eine fotooxidative Zerstörung der Proteine jedoch nicht bewirken kann. Da als Trägermaterial sehr häufig Röhrchen verwendet werden, sollte die Wellenlänge darüberhinaus bevorzugt in einem Bereich liegen, in dem eine Bestrahlung innerhalb der gefüllten Röhrchen durchgeführt werden kann. Limitierend ist hierbei die Lichtdurchlässigkeit der Röhrchen.

Die Bestrahlungsdauer richtet sich nach der Reaktivität und Lichtempfindlichkeit der fotoaktivierbaren Gruppen und liegt in der Regel zwischen einer Sekunde und einer Stunde. Besonders bevorzugt beträgt die Bestrahlungszeit 30 bis 60 Minuten.

Zur Bestrahlung können an sich bekannte Vorrichtungen verwendet werden. Dabei sollte darauf geachtet werden, daß eine homogene Bestrahlung der Röhrchen gewährleistet ist, da Inhomogenitäten zu Schwankungen bei der Wandhaftung führen.

Mit dem erfindungsgemäßen Verfahren gelingt es, Trägermaterial mit immunologisch aktiver Substanz zu beschichten, ohne daß eine Vorbehandlung der Substanz oder der Oberflächen notwendig ist. Das Verfahren ist ohne großen Aufwand in übliche Produktionsprozesse integrierbar, da nur ein zusätzlicher Einsatzstoff für die Beladelösungen und eine online-Bestrahlung des Trägermaterials erforderlich ist. Es ist universell anwendbar und eignet sich für die Fixierung der unterschiedlichsten Substanzen auf vielen verschiedenen verfügbaren Trägermaterialien. Das Verfahren führt zu einer deutlichen Verbesserung der Wandhaftung. Die Ablöseraten der Wandkomponenten während des Funktionstestes und unter Temperatur- und Detergensbelastung werden wesentlich reduziert. Die Eichkurven, Wiederfindungen und Präzisionen bleiben gegenüber bekannten beschichteten Röhrchen unverändert.

Die Erfindung wird durch die folgenden Figuren und Beispiele erläutert:

Die Figuren 1 bis 5 zeigen Diagramme, in denen Ablösedaten für beschichtete Tubes, die nach dem erfindungsgemäßen Verfahren beschichtet wurden und für Tubes, die adsorptiv beschichtet wurden (im weiteren als "normal beschichtete Tubes" bezeichnet), aufgetragen sind:

Fig. 1 Eichkurven für Prolactin-Test bei 20°C, $\lambda$=405 nm
Kurve 1: normal beladene Tubes
Kurve 2: fotofixierte Tubes;

Fig. 2 Eichkurven für Prolactin-Test bei 37°C $\lambda$=405 nm
Kurve 1: normal beladene Tubes
Kurve 2: fotofixierte Tubes

Fig. 3 Eichkurven für 1H-Test bei 20°C, $\lambda$=405 nm
Kurve 1: normal beladene Tubes (Luran)

Kurve 2: fotofixierte Tubes

Fig. 4 Eichkurven für $T_4$-Test bei 20°C, λ=405 nm

Kurve 1: normal beladene Tubes (1 µg/ml)

Kurve 2: fotofixierte Tubes (1 µg/ml)

Kurve 3: fotofixierte Tubes (1 µg/ml)

Fig. 5 Eichkurven für $T_3$-Test bei 30°C, λ=405 nm

Kurve 1: normal beladene γ-Tubes

Kurve 2: normal beladene Polystyrol-Tubes

Kurve 3: fotofixierte Polystyrol-Tubes

Kurve 4: fotofixierte γ-Tubes.

**Beispiel 1**

Nach dem erfindungsgemäßen Verfahren wurden Tubes mit verschiedenen immunologisch aktiven Substanzen beschichtet. Alle eingesetzten Antikörper, Polyhaptene und Bindeproteine wurden radioaktiv mit 125 J markiert. Mittels radiometrischer Messungen wurden bestimmt:

- die an die Festphase gebundene Menge an immunologisch aktiver Substanz (Differenz der Nutzraten von mit Beladelösung gefüllten und nach Abschluß der Beladung leergesaugten Tubes),
- die Ablösung der Wandkomponenten während des Funktionstestes (bei verschiedenen Temperaturen) sowie unter Detergenzbelastung. Die beschichteten Tubes wurden hierzu vor und nach Durchführung des Funktionstestes bzw. vor und nach der Inkubation mit detergenshaltigen Puffern (40 mmol/l Natriumphosphat, pH 7,4) in einem γ-Counter vermessen. Aus der Differenz der gemessenen Nutzraten wurde die prozentuale Ablösung errechnet.

Zur weiteren Beurteilung wurden die beschichteten Tubes in Funktionstesten eingesetzt. Prüfkriterien waren Eichkurven, Präzisionen, Wiederfindungen und die Tube-Stabilitäten. Als Referenz bei allen Messungen diente jeweils eine normal, d.h. adsorptiv beschichtete Tube-Variante.

a) adsorptive Beschichtung:

Die immunologisch aktive Substanz (0,7 bis 10 µg/ml) wird in 40 mmol/l Phosphatpuffer, pH 7,4 (bzw. Puffer I für den $T_4$-Test, vgl. Beispiel 4; Puffer II für den TSH-Test, vgl. Beispiel 6; Puffer III für Streptavidin, vgl. Beispiel 7) 24 Stunden bei Raumtemperatur inkubiert. Anschließend wird wie unter Punkt b)2. von Beispiel 1 beschrieben nachbeladen und getrocknet.

b) Fotobeschichtung (Fotofixierung):

Die Fotobeschichtung erfolgte wie die adsorptive Beschichtung mit folgenden Modifikationen:

1. die Beladelösung enthielt einen Zusatz von 1,5 % Ethanol und $1 \cdot 10^{-5}$ mol/l 4,4′-Dithio-bis-phenylazid. Die Konzentration der immunologisch aktiven Substanzen (0,7 bis 16 µg/ml) und die Pufferkonzentration entsprachen der Zusammensetzung der Beladelösung für die adsorptive Beladung. Die modifizierte Beladelösung wurde in Polystyrol-Tubes, γ-bestrahlten Polystyrol-Tubes (γ-Tubes) oder Luran-Tubes 20 bis 24 Stunden bei 20°C im Dunkeln inkubiert.
2. Nach Beendigung des ersten Inkubationsschrittes wurden die noch mit der Erstbeladelösung gefüllten Tubes für eine Stunde unter UV-Licht mit einer Wellenlänge von 366 nm bestrahlt. Anschließend erfolgt eine Nachbeladung (0,3%ige RSA-Lösung mit 0,9 % Natriumchlorid und 2 % Saccharose, 30 min bei 20°C) und Trocknung (24 Stunden bei 20°C und 40 % relativer Feuchte) der Tubes.

**Beispiel 2**

Nach den Angaben von Beispiel 1 wurden Teströhrchen für einen Prolactin-Test hergestellt. Die Beladung erfolgte mit 4 µg/ml Antikörper und $1 \cdot 10^{-5}$ mmol/l 4,4′-Dithio-bis-phenylazid (DPA) in 40 mmol/l Phosphatpuffer, pH 7,4. Die Bestrahlung erfolgte 60 Minuten bei 366 nm. Die mit diesen Röhrchen erhaltenen Ergebnisse im Vergleich zu normal beschichteten Röhrchen sind den Figuren 1 und 2 sowie den Tabellen 1 und 2 zu entnehmen. Es zeigt sich, daß mit den fotofixierten Röhrchen stark reduzierte Ablöseraten auch unter Detergens- und Temperaturbelastung gefunden werden bei vergleichbaren Eichkurven, Präzisionen und Wiederfindungen. Zur Durchführung des Funktionstestes wurden 100 mU eines POD-markierten monoklonalen Antikörpers gegen Prolactin in 1 ml Puffer (40 mmol/l Natriumdihydrogenphosphat, pH 7,4) zusammen mit 50 µl Probe oder Standard für 30 Minuten bei 20 und 37°C in den beschichteten Tubes inkubiert. Nach zweimaligem Waschen mit Leitungswasser wurde 1 ml ABTS® Substratlösung (1,9 mmol/l 2,2′-Azinodi-[3-ethyl-benzthiazolin-sulfonsäure(6)]-diammoniumsalz, 100 mmol/l Phosphat-Citratpuffer, pH 4,4, 3,2 mmol/l Natriumperborat) hinzugegeben und nach 30 Minuten die Extinktion bei 405 nm gemessen.

Tabelle 1

| | | Normal beladene Tubes [1] | foto-fixierte Tubes [1] |
|---|---|---|---|
| Gebundene Proteinmenge (µg AK/Tube) | | 1,45 | 1,17 |
| Variationskoeffizient (n = 8) | | 1,9 % | 2,5 % |
| Wiederfindung von Humanseren (n = 5) | Serum 1 | 100 % | 102 % |
| | Serum 2 | 100 % | 104 % |
| | Serum 3 | 100 % | 100 % |
| Ak-Ablösung während Funktionstest | bei 20°C | 6,2 % | 1,6 % |
| | bei 37°C | 23,0 % | 2,2 % |
| AK-Ablösung bei Detergenzbelastung (30 min, RT) | 1% Pluronic | 8,3 % | 2,3 % |
| | 1% SDS | 45,0 % | 2,8 % |
| | 0,5% Triton | 80,0 % | 3,5 % |

n: Anzahl der Bestimmungen
SDS: Natriumdodecylsulfat
RT: Raumtemperatur
Pluronic: Pluronic F68 (BASF), Blockpolymeres aus Ethylen- und Propylenoxid
Triton: Triton® x 100 (Rohm und Haas), Ethoxylate des 4-(1,1,3,3-Tetramethylbutyl)phenols
[1] aus Luran

Tabelle 2

| | | Normal beladene Tubes 2) | | fotofixierte Tubes 2) | |
|---|---|---|---|---|---|
| | | unbelastet 3 Wo/4°C (Referenz) | belastet 3 Wo/37°C | unbelastet 3Wo/4°C (Referenz) | belastet 3 Wo/37°C |
| Präzision 1) (n = 3) | Serum 1 x = 0,065 | 9,4 % | 5,2 % | 7,8 % | 2,7 % |
| | Serum 2 x = 0,06 | 3,6 % | 1,0 % | 6,1 % | 4,2 % |
| | Serum 3 x=0,165 | 1,6 % | 1,6 % | 3,6 % | 1,3 % |
| Wiederfindung von Humanseren (n = 3) | Serum 1 | 100 % | 100 % | 100 % | 95 % |
| | Serum 2 | 100 % | 105 % | 100 % | 92 % |
| | Serum 3 | 100 % | 80 % | 100 % | 109 % |
| Wiederfindung von Standards n = 3 | Std.* a | 100 % | 112 % | 100 % | 107 % |
| | Std. c | 100 % | 92 % | 100 % | 95 % |
| | Std. e | 100 % | 93 % | 100 % | 95 % |

* Std. = Standard
X: mittlere Extinktion,
1) bezogen auf Extinktionswerte
2) aus Luran

**Beispiel 3**

Es wurden Röhrchen für einen LH-Test beschichtet. Die Bedingungen waren, wie im Beispiel 1 beschrieben. Die Beladung erfolgte mit 1,5 µg/ml Antikörper in 40 mmol/l Phosphatpuffer, pH 7,2. Die Ergebnisse mit diesen Röhrchen sind der Tabelle 3 sowie der Fig. 3 zu entnehmen. Es zeigt sich, daß Temperatur- und Detergensstabilität ausgezeichnet sind bei vergleichbaren Eichkurven, Präzisionen und Wiederfindungen. Die Menge an gebundenen Antikörpern liegt im selben Bereich wie bei normal beschichteten Röhrchen.

Zur Durchführung des Funktionstestes wurden 66 mU eines POD-markierten monoklonalen Antikörpers gegen 1H in 1 ml Puffer (40 mmol/l Phosphat, pH 7,4) zusammen mit 100 µl Probe oder Standard für 2 Stunden bei 20 und 30°C inkubiert. Nach dreimaligem Waschen mit Leitungswasser wurde 1 ml ABTS® Substratlösung hinzugegeben und nach 30 min bei 20°C die Extinktion bei 405 nm gemessen.

Tabelle 3

| | | Normal beladene Tubes (Luran) | Normal beladene γ-Tubes | Foto-fixierte γ-Tubes |
|---|---|---|---|---|
| Gebundene Proteinmenge (μg AK/Tube) | | 1,05 | 1,30 | 1,12 |
| AK-Ablösung bei Detergensbelastung (30 min RT) | 1% Pluronic (n = 10) | 3,1 % | 5,3 % | 1,3 % |
| | 0,5 % Triton (n = 10) | 78,0 % | 15,1 % | 11,0 % |
| AK-Ablösung während Funktionstest bei 20°C (n = 10) | Puffer-Standards | 3,1 % | 6,5 % | 1,6 % |
| | Serum-Standards | 3,3 % | 6,8 % | 1,8 % |
| | Humanseren | 3,4 % | 6,6 % | 1,7 % |
| AK-Ablösung während Funktionstest bei 30°C (n = 10) | Puffer-Standards | 9,6 % | 11,0 % | 3,2 % |
| | Serum-Standards | 10,6 % | 9,6 % | 2,9 % |
| | Humanseren | 9,7 % | 12,4 % | 2.9 % |
| Wiederfindung von Humanseren (n = 3) | Serum 1 | 100 % | 66 % | 92 % |
| | Serum 2 | 100 % | 97 % | 109 % |
| | Serum 3 | 100 % | 92 % | 95 % |
| | Serum 4 | 100 % | 86 % | 92 % |
| | Serum 5 | 100 % | 96 % | 98 % |
| Präzision [1] (n = 3) | Serum 1 | 14,9% | 7,4% | 12,9% |
| | Serum 2 | 2,2% | 2,3% | 3,1% |
| | Serum 3 | 5,2% | 7,4% | 5,5% |
| | Serum 4 | 6,9% | 4,6% | 1,6% |
| | Serum 5 | 4,3% | 4,0% | 3,2% |

[1] bezogen auf die Extinktionswerte

**Beispiel 4**

Es wurden Röhrchen beschichtet für einen T4-Test. Es wurden zwei Chargen gebildet, wobei für die erste

Charge eine Beladelösung mit 1 µg/ml Antikörper und 2,5 µg/ml RSA Phosphatpuffer, pH 7,4 und für die zweite Charge eine Beladelösung mit 2 µg/ml Antikörper und 2,5 µg/ml RSA Phosphatpuffer pH 7,4 verwendet wurde. Die sonstigen Beschichtungsbedingungen waren wie im Beispiel 1 beschrieben. Die Ergebnisse sind den Tabellen 4, 5 und 6 sowie der Fig. 4 zu entnehmen. Es wurden wiederum Röhrchen mit sehr guter Tube-Stabilität und reduzierten Ablöseraten beim Funktionstest und bei Detergensbelastung erhalten. Die Präzision war insgesamt sehr gut und die Wiederfindungswerte lagen im Bereich der normal beschichteten Röhrchen.

## Tabelle 4

| | | Normal beladene Tubes 2) | foto-fixierte Tubes 2) |
|---|---|---|---|
| Präzision 1) (n = 30) | Serie 1 | 6,2 % | 2,8 % |
| | Serie 2 | 5,9 % | 3,2 % |
| | Serie 3 | 5,3 % | 2,7 % |
| Wiederfindung von Humanseren (n = 5) | Serum 1 | 100 % | 101 % |
| | Serum 2 | 100 % | 108 % |
| | Serum 3 | 100 % | 100 % |
| AK-Ablösung während Funktionstest (n = 30) | Serie 1 | 9,1 % | 3,2 % |
| | Serie 2 | 8,9 % | 3,2 % |
| | Serie 3 | 8,1 % | 2,8 % |
| AK-Ablösung bei Detergenzbelastung (30 min, RT) | 1% Pluronic | 11,0 % | 2,8 % |
| | 1 % SDS | 44,0 % | 12,0 % |
| | 0,5% Triton | 86,0 % | 16,0 % |

1) bezogen auf die Extinktionswerte
2) aus Polystyrol

Zur Durchführung des Funktionstests wurden 15 mU eines T4-POD-Konjugates in 1 ml Puffer I (120 mmol/l Na-Barbiturat, 18,2 mmol/l Phosphat, 0,04 % ANS (8-Anilino-1-naphthalinsulfonsäure), pH 8,6) zusammen mit 20 µl Probe oder Standard für 30 Minuten bei 20°C in den Tubes inkubiert. Nach einmaligem Waschen mit Leitungswasser wurde 1 ml ABTS®-Substratlösung hinzugegeben und nach 30 min (20°C) die Extinktion bei 405 nm gemessen.

Tabelle 5

| | Normal beladene Tubes [3] 1μg AK/ 2,5 μg RSA | fotofixierte Tubes [3] 1 g AK/2,5 g RSA | fotofixierte Tubes [3] 2 g AK/2,5 g RSA |
|---|---|---|---|
| gebundene AK-Menge (μg/Tube) n = 15 | 0,425 (100 %) | 0,352 (83 %) | 0,70 (165 %) |
| Funktions-signal Stand. a (E) n = 15 | 1,253 (100 %) | 0,927 (74 %) | 1,466 (120 %) |
| spez. Reaktivität ΔE/μg Wand-AK n = 15 | 2,9 (100 %) | 2,6 (89 %) | 2,1 (72 %) |
| AK-Ablösung im [2] Funktionstest (n = 15) | 11,8 % | 2,6 % | 2,8 % |
| AK-Ablösung bei Detergenz-belastung [1] (n = 15) | 92,4 % | 3,8 % | 4,2 % |

1) 0,5 % Triton 30 min, RT
2) bei 20°C
3) aus Polystyrol

Tabelle 6

| | | Normal beladene Tubes 2) | | fotofixierte Tubes 2) | |
|---|---|---|---|---|---|
| | | unbelastet 3 Wo/4°C (Referenz) | belastet 3 Wo/ 37°C | unbelastet 3Wo/4°C (Referenz) | belastet 3 Wo/ 37°C |
| Präzision (n = 3) Extinktionsbasis | Serum 1 x=0,659 | 1,7 % | 2,3 % | 1,8 % | 1,3 % |
| | Serum 2 x=0,653 | 1,7 % | 0,8 % | 2,3 % | 2,8 |
| | Serum 3 x=0,854 | 1,7 % | 0,8 % | 3,0 % | 1,1 % |
| Wiederfindung von Humanseren (n = 3) | Serum 1 | 100 % | 99 % | 100 % | 104 % |
| | Serum 2 | 100 % | 104 % | 100 % | 100 % |
| | Serum 3 | 100 % | 101 % | 100 % | 101 % |
| Wiederfindung Standards 1) n = 3 | Std.* a | 100 % | 102 % | 100 % | 99 % |
| | Std.* b | 100 % | 102 % | 100 % | 103 % |
| | Std.* d | 100 % | 100 % | 100 % | 98 % |

* Std. = Standard

1) bezogen auf Extinktionswerte

2) aus Polystyrol

**Beispiel 5**

Es wurden Röhrchen für einen T3-Polyhaptentest beschichtet. Dazu wurden die Röhrchen mit einer Lösung, die 0,75 µg/ml Polyhapten (PH)/2 µg/ml R-IgG in 40 mmol/l Phosphatpuffer, pH 7,4 enthielt, inkubiert. Die übrigen Bedingungen waren, wie im Beispiel 1 beschrieben. Die Ergebnisse mit diesen Röhrchen sind der folgenden Tabelle 7 sowie der Figur 5 zu entnehmen. Die Fotofixierung führt auch hier zu deutlich reduzierten Ablöseraten gegenüber normal beschichteten Röhrchen. Die Beschichtung ist gegenüber starken Detergentien und erhöhten Temperaturen stabil.

Zur Durchführung des Funktionstestes wurden 80 mU eines POD-markierten Antikörpers gegen T3 in 1 ml Puffer II (120 mmol/l Barbitalpuffer, 0,04 % ANS) zusammen mit 200 µl Probe oder Standard für 30 min bei 20 und 30°C in den Tubes inkubiert. Nach zweimaligem Waschen mit Leitungswasser wurde 1 ml ABTS®-Substratlösung hinzugegeben und nach 30 min bei 20°C die Extinktion bei 405 nm gemessen.

Tabelle 7

| | | Normal beladene PS-Tubes | Normal beladene ɣ-Tubes | fotofixierte PS-Tubes | fotofixierte ɣ-Tubes |
|---|---|---|---|---|---|
| Gebundene PH-Menge (µg/Tube) n=10 | | 0,73 | 0,82 | 0,62 | 0,66 |
| Funktionssignal Standard a (E) n=5 | 20°C | 1,24 | 1,16 | 0,7 | 0,9 |
| | 30°C | 1,83 | 1,98 | 1,34 | 1,72 |
| spezifische Reaktivität E/µg Wand-PH n=5 | 20°C | 1,69 | 1,41 | 1,12 | 1,36 |
| | 30°C | 2,5 | 2,4 | 2,2 | 2,6 |
| PH-Ablösung während Funktionstest n=10 | 20°C | 14,2 % | 12,5 % | 2,7 % | 2,9 % |
| | 30°C | 23,3 % | 17,7 % | 3,6 % | 3,6 % |
| PH-Ablösung bei Detergenzbelastung 0,5 % Triton, 20 min RT, n=10 | | 74,2 % | 21,2 % | 1,7 % | 2,2 % |

**Beispiel 6**

Es wurden Röhrchen für einen TSH-Test beschichtet. Dazu wurden die Röhrchen unter den in Beispiel 1 beschriebenen Bedingungen mit einer Beladelösung mit 2 µg/ml Antikörper (AK) gegen TSH inkubiert. Eine Überprüfung der Ablöseraten ergab die in der folgenden Tabelle 8 aufgeführten Ergebnisse.

Tabelle 8

Ablösung der Wandkomponente durch Detergenzien

| | | Normal beladene Tubes | fotofixierte Tubes |
|---|---|---|---|
| Gebundene Proteinmenge (μg/Tube) n=10 | | 0,87 | 0,87 |
| AK-Ablösung während Funktionstest bei RT (n=5) | Serie 1 | 3,8 % | 2,5 % |
| | Serie 2 | 3,2 % | 2,9 % |
| | Serie 3 | 4,1 % | 2,4 % |
| | Serie 4 | 4,3 % | 2,9 % |
| | Serie 5 | 3,7 % | 3,3 % |
| AK-Ablösung bei Detergenzbelastung (0,5 % Triton, 30 min RT) (n=5) | Serie 1 | 75,4 % | 1,9 % |
| | Serie 2 | 72,1 % | 1,8 % |
| | Serie 3 | 74,3 % | 1,8 % |
| | Serie 4 | 75,0 % | 2,3 % |
| | Serie 5 | 79,7 % | 2,0 % |

**Beispiel 7**

Es wurden mit Streptavidin und Thermo-RSA-Streptavidin (TRS) beschichtete Röhrchen nach EP-A 0 269 092 hergestellt. Für die erste Charge wurden die Röhrchen unter den im Beispiel 1 beschriebenen Bedingungen mit einer 4 μg/ml Streptavidin enthaltenden Lösung 40 mmol/l Phosphatpuffer, pH 7,4, inkubiert. In einer zweiten Charge wurden Röhrchen mit Beladelösungen, die 4 bzw. 16 μg/ml TRS enthielten, inkubiert. Die Röhrchen wurden hinsichtlich der Wandhaftung bzw. der Biotin-Bindekapazität (BiKa) getestet. Zur Bestimmung der relativen Biotin-Bindekapazität wurden 18 ng Biotin und 70 mU Biotin-POD-Konjugat in 1,2 ml Puffer III (40 mmol/l Phosphat, pH 7,0, 0,5 % Pluronic F68) für 30 min in den Tubes inkubiert. Nach einmaligen Waschen (5 min) mit Leitungswasser wurden 1 ml ABTS® Substratlösung hinzugegeben und nach 30 min die Extinktion bei 405 nm gemessen. Die Ergebnisse sind den folgenden Tabellen 9 bis 11 zu entnehmen. Es zeigt sich, daß die Fotofixierung von Streptavidin und TRS zu teilweise deutlicher Verbesserung der Wandhaftung führt.

## Tabelle 9

Ablösung der Wandkomponente in Phosphatpuffer mit 2 % Pluronic (Inkubationszeit 30 Minuten bei 20 und 30°C)

a) Streptavidin-Tubes

| Tubevariante | % Ablösung 20°C | % Ablösung 30°C |
|---|---|---|
| Luran Tubes | 17,8 | 28,0 |
| Luran Tubes, fotofixiert | 3,1 | 5,7 |

n = 5

b) Thermo-RSA-Streptavidin Tubes

| Tubevariante | % Ablösung 20°C | % Ablösung 30°C |
|---|---|---|
| Luran Tubes | 3,1 | 11,6 |
| Luran Tubes, fotofixiert | 0,8 | 1,7 |

Tabelle 10

a) Streptavidin Tubes

| Tubevariante | % Ablösung 1% Pluronic F68 (30min, RT) | % Ablösung 0,5 % Triton (30min, RT) |
|---|---|---|
| Luran Tubes | 13,9 | 42 |
| Luran Tubes, fotofixiert | 3,3 | 9,4 |

b) Thermo-RSA-Streptavidin Tubes

| Tubevariante | % Ablösung 1% Pluronic F68 (30min, RT) | % Ablösung 0,5 % Triton (30min, RT) |
|---|---|---|
| Luran Tubes | 2,1 | 8,0 |
| Luran Tubes, fotofixiert | 0,9 | 0,8 |

n = 5

Tabelle 11

| | Normal beschichtete Tubes | | | | Fotofixierte Tubes | | | |
|---|---|---|---|---|---|---|---|---|
| | Beladekonzentration 4 µg/ml | | Beladekonzentration 16 µg/ml | | Beladekonzentration 4 µg/ml | | Beladekonzentration 16 µg/ml | |
| | Lu | ɣ | Lu | ɣ | Lu | ɣ | Lu | ɣ |
| Gebundene TRS-Menge µg/Tube | 3,6 | 3,78 | 5,05 | 5,26 | 3,05 | 3,06 | 4,57 | 4,73 |
| relative Bika (Extinktionen) | 0,68 | 0,7 | 1,16 | 1,16 | 0,25 | 0,21 | 0,39 | 0,28 |
| TRS-Ablösung während Bika-Test | 0,8% | 1,8% | 2,0% | 1,7% | 1,6% | 1,3% | 2,1% | 1,9% |
| TRS-Ablösung 1% Pluronic 30 min, RT | 3,3% | 2,9% | 1,6% | 0,4% | 1,0% | 0,6% | 1,2% | 1,2% |
| TRS-Ablösung 0,5 % Triton 30 min, RT | 18,6% | 1,3% | 21,5% | 1,5% | 1,1% | 0,6% | 1,2% | 1,2% |

n = 5

## Patentansprüche

1. Verfahren zur Herstellung einer mit einer immunologischaktiven Substanz beschichteten Festphasenmatrix,
**dadurch gekennzeichnet**,
daß man ein Trägermaterial mit einer Mischung aus
(a) immunologisch aktiver Substanz,
(b) einer Verbindung, die mindestens zwei photoaktivierbare funktionelle Gruppen und mindestens einen mit dem Trägermaterial und der immunologisch aktiven Substanz adsorptiv wechselwirkenden hydrophoben Anteil enthält,
absorptiv beschichtet und anschließend zur Vernetzung bestrahlt.

2. Verfahren nach Anspruch 1, **dadurch gekennzeichnet**, daß man als immunologisch aktive Substanz Antikörper, Antigene oder ein Bindeprotein verwendet.

3. Verfahren nach einem der vorhergehenden Ansprüche, **dadurch gekennzeichnet**, daß man immunologisch aktive Substanz und fotoaktivierbare Verbindung in einem molaren Verhältnis von 1:1 bis 1:1x10$^6$ einsetzt.

4. Verfahren nach einem der vorhergehenden Ansprüche, **dadurch gekennzeichnet,** daß man als immunologisch aktive Substanz Anti-T4-Antikörper, Anti -Prolactin-Antikörper, Anti-LH-Antikörper, Anti-TSH-Antikörper, T3-Polyhapten, Streptavidin, Thermo-Rinderserumalbumin(RSA)-Streptavidin und/oder poly-Streptavidin verwendet.

5. Verfahren nach einem der vorhergehenden Ansprüche, **dadurch gekennzeichnet**, daß man als Komponente (b) eine homo-bifunktionelle Verbindung verwendet.

6. Verfahren nach einem der vorhergehenden Ansprüche, **dadurch gekennzeichnet**, daß man als Komponente (b) eine Verbindung der allgemeinen Formel

V-Hy-W

verwendet, worin V und W jeweils eine fotoaktivierbare funktionelle Gruppe und Hy eine hydrophobe Gruppe bedeuten.

7. Verfahren nach einem der vorhergehenden Ansprüche, **dadurch gekennzeichnet**, daß man als Komponente (b) eine Verbindung der folgenden Formel:

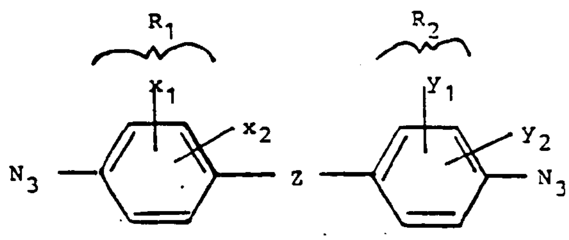

worin $R_1$ und $R_2$ die hydrophoben Anteile bezeichnen und $x_1$, $x_2$ und $y_1$, $y_2$ jeweils unabhängig voneinander H, $NO_2$ oder Halogen und Z eine Spacer-Gruppe bedeuten, verwendet.

8. Verfahren nach einem der vorhergehenden Ansprüche, **dadurch gekennzeichnet**, daß man als Trägermaterial Kunststoff, Glas, Polysaccharide, Keramik oder Cellulose verwendet.

9. Verfahren nach einem der vorhergehenden Ansprüche, **dadurch gekennzeichnet**, daß man die Bestrahlung bei einer Wellenlänge von 250 bis 500 nm durchführt.

10. Verfahren nach einem der vorhergehenden Ansprüche, **dadurch gekennzeichnet**, daß man die beschichtete Festphasenmatrix 0,5 bis 1 Stunde bestrahlt.

## Claims

1. Process for the production of a solid phase matrix coated with an immunologically active substance, wherein a carrier material is coated absorptively with a mixture of
   (a) an immunologically active substance,
   (b) a compound which contains at least two functional groups which can be photoactivated and at least one hydrophobic moiety which interacts adsorptively with the carrier material and with the immunologically active substance,
   and is subsequently irradiated for the cross-linkage.

2. Process as claimed in claim 1, wherein antibodies, antigens or a binding protein is used as the immunologically active substance.

3. Process as claimed in one of the previous claims, wherein the immunologically active substance and the compound which can be photoactivated are used in a molar ratio of 1:1 to $1:1x10^6$.

4. Process as claimed in one of the previous claims, wherein anti-T4 antibodies, anti-prolactin antibodies, anti-LH antibodies, anti-TSH antibodies, T3-polyhapten, streptavidin, thermo-bovine-serum-albumin (BSA)-streptavidin and/or polystreptavidin are used as the immunologically active substance.

5. Process as claimed in one of the previous claims, wherein a homo-bifunctional compound is used as the component (b).

6. Process as claimed in one of the previous claims, wherein a compound of the general formula

V-Hy-W

is used as the component (b) in which V and W each denote a functional group which can be photoactivated and Hy denotes a hydrophobic group.

**7.** Process as claimed in one of the previous claims, wherein a compound of the following formula:

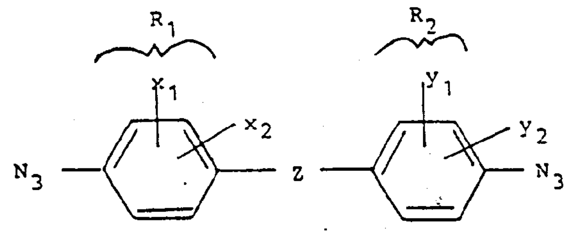

is used as the component (b) in which $R_1$ and $R_2$ denote the hydrophobic moieties and $x_1$, $x_2$ and $y_1$, $y_2$ denote H, $NO_2$ or halogen, in each case independently from each other, and Z denotes a spacer group.

**8.** Process as claimed in one of the previous claims, wherein plastic, glass, polysaccharides, ceramics or cellulose are used as the carrier material.

**9.** Process as claimed in one of the previous claims, wherein the irradiation is carried out at a wavelength of 250 to 500 nm.

**10.** Process as claimed in one of the previous claims, wherein the coated solid phase matrix is irradiated for 0.5 to 1 hour.

## Revendications

**1.** Procédé de préparation d'une matrice en phase solide revêtue d'une substance immunologiquement active, caractérisé en ce que l'on revêt par absorption un matériau support d'un mélange

(a) de la substance immunologiquement active,

(b) d'un composé qui contient au moins deux groupes fonctionnels photo-activables et au moins une partie hydrophobe interagissant par adsorption avec le matériau support et la substance immunologiquement active,

puis on l'irradie pour la réticulation.

**2.** Procédé selon la revendication 1, caractérisé en ce que l'on utilise comme substance immunologiquement active des anticorps, des antigènes ou une protéine de liaison.

**3.** Procédé selon l'une des revendications précédentes, caractérisé en ce que l'on utilise la substance immunologiquement active et le composé photo-activable dans un rapport molaire de 1:1 à $1:1x10^6$.

**4.** Procédé selon l'une des revendications précédentes, caractérisé en ce que l'on utilise comme substance immunologiquement active un anticorps anti-T4, un anticorps anti-prolactine, un anticorps anti-LH, un anticorps anti-TSH, un polyhaptène T3, la streptavidine, la thermosérumalbumine bovine(SAB)-streptavidine et/ou la polystreptavidine.

**5.** Procédé selon l'une des revendications précédentes, caractérisé en ce que l'on utilise comme constituant (b) un composé homobifonctionnel.

**6.** Procédé selon l'une des revendications précédentes, caractérisé en ce que l'on utilise comme constituant (b) un composé de formule générale

V-Hy-W

dans laquelle V et W représentent chacun un groupe fonctionnel photo-activable et Hy représente un groupe hydrophobe.

7. Procédé selon l'une des revendications précédentes, caractérisé en ce que l'on utilise comme constituant (b) un composé de formule suivante:

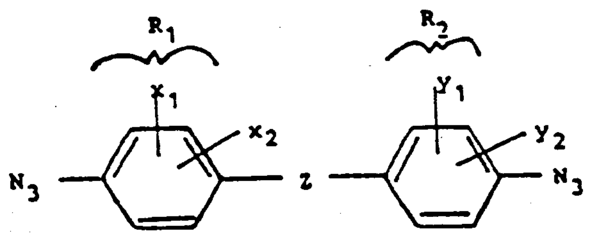

dans laquelle $R_1$ et $R_2$ représentent les parties hydrophobes et $x_1$, $x_2$ et $y_1$, $y_2$ représentent chacun indépendamment les uns des autres H, $NO_2$ ou halogène et Z représente un groupe espaceur.

8. Procédé selon l'une des revendications précédentes, caractérisé en ce que l'on utilise comme matériau support une matière synthétique, le verre, des polysaccharides, une céramique ou la cellulose.

9. Procédé selon l'une des revendications précédentes, caractérisé en ce que l'on conduit l'irradiation à une longueur d'onde de 250 à 500 nm.

10. Procédé selon l'une des revendications précédentes, caractérisé en ce que l'on irradie pendant 0,5 à 1 heure la matrice en phase solide revêtue.

FIG.1

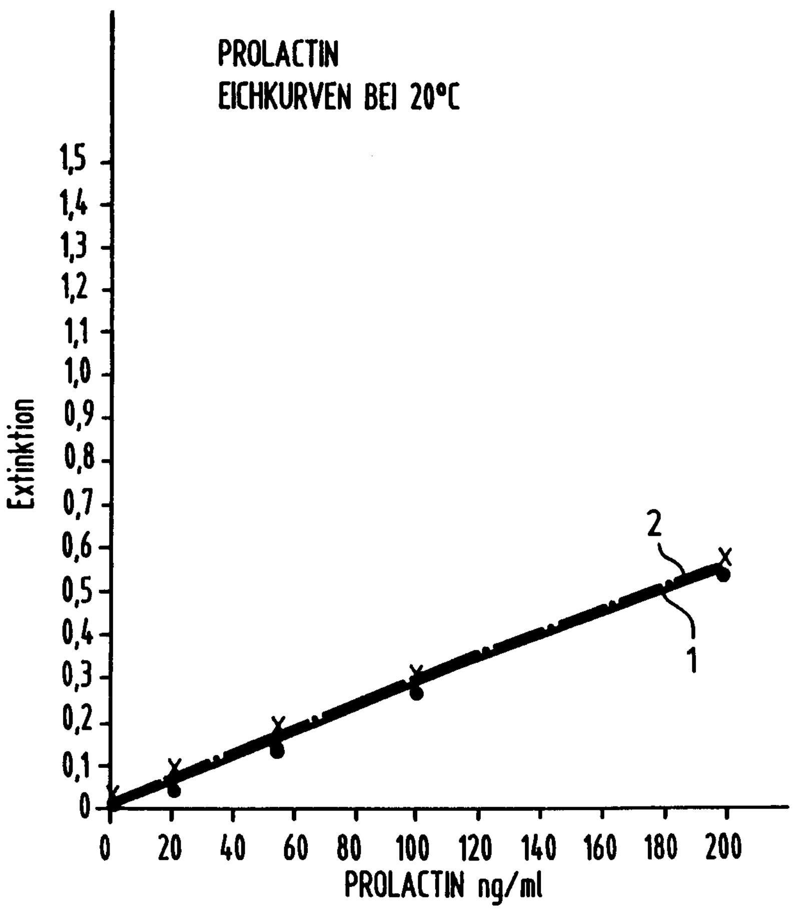


Kurve 1 : normal beladene Tubes
Kurve 2 : photofixierte Tubes

FIG.2

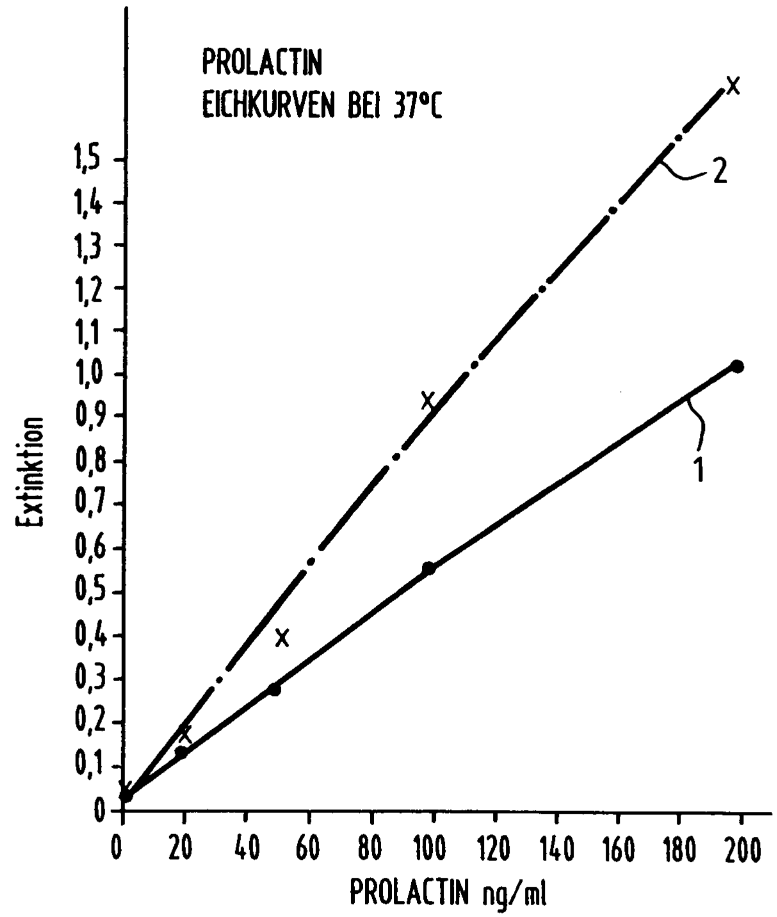


Kurve 1 : normal beladene Tubes
Kurve 2 : photofixierte Tubes

FIG.3

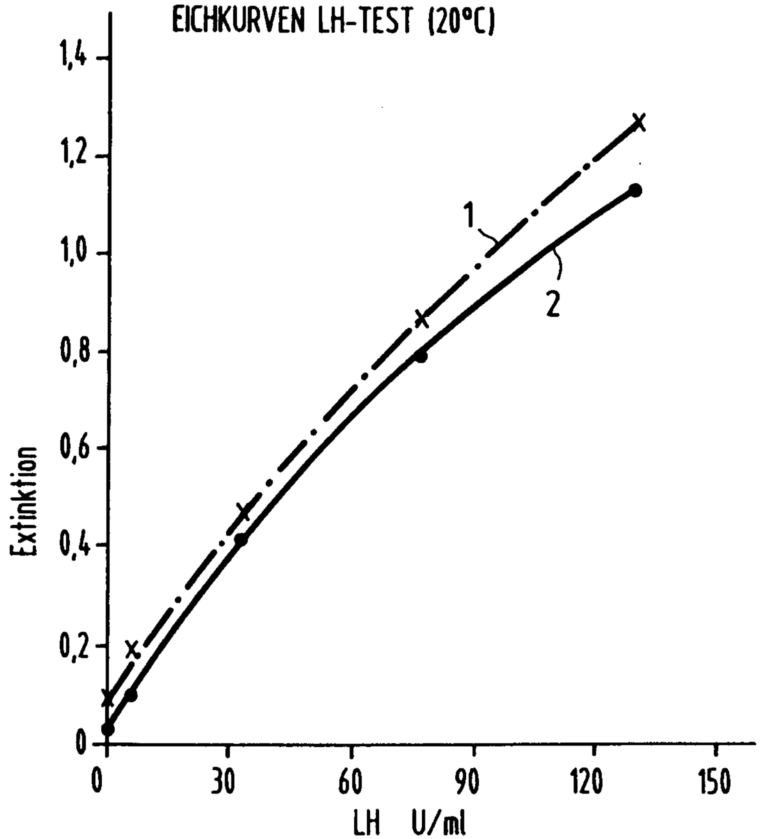


Kurve 1 : normal beladene Tubes (Luran)
Kurve 2 : photofixierte Tubes

FIG.4

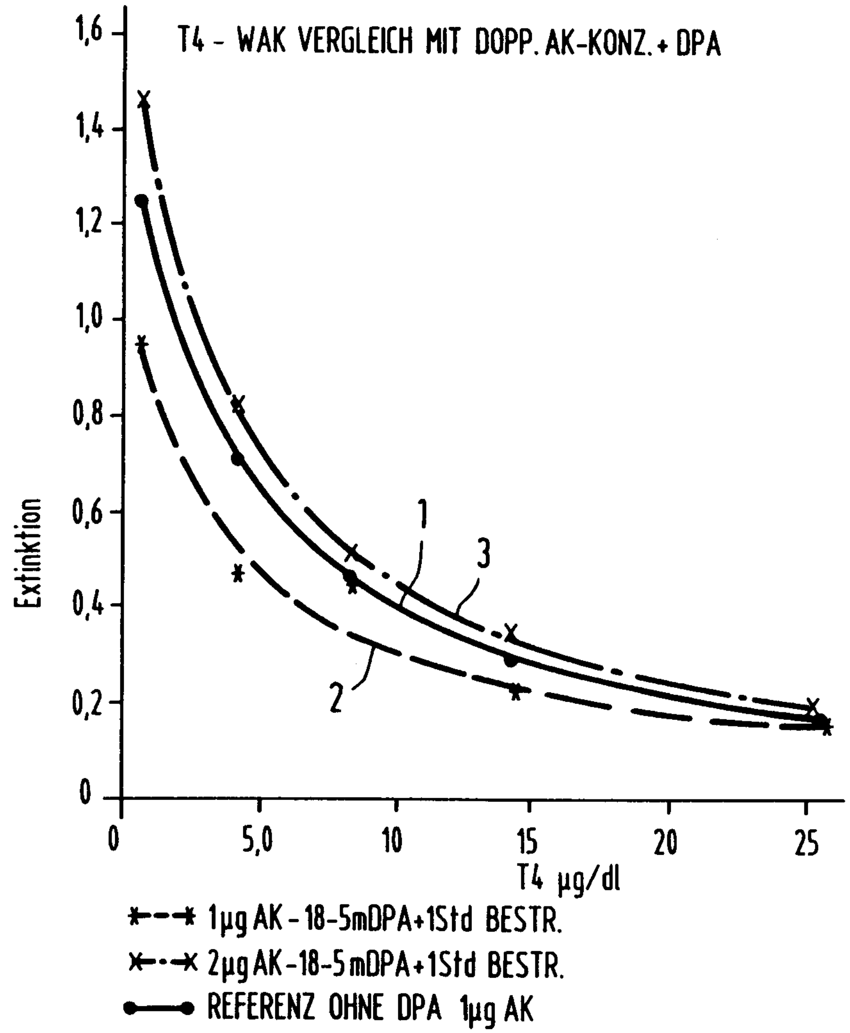

FIG. 5

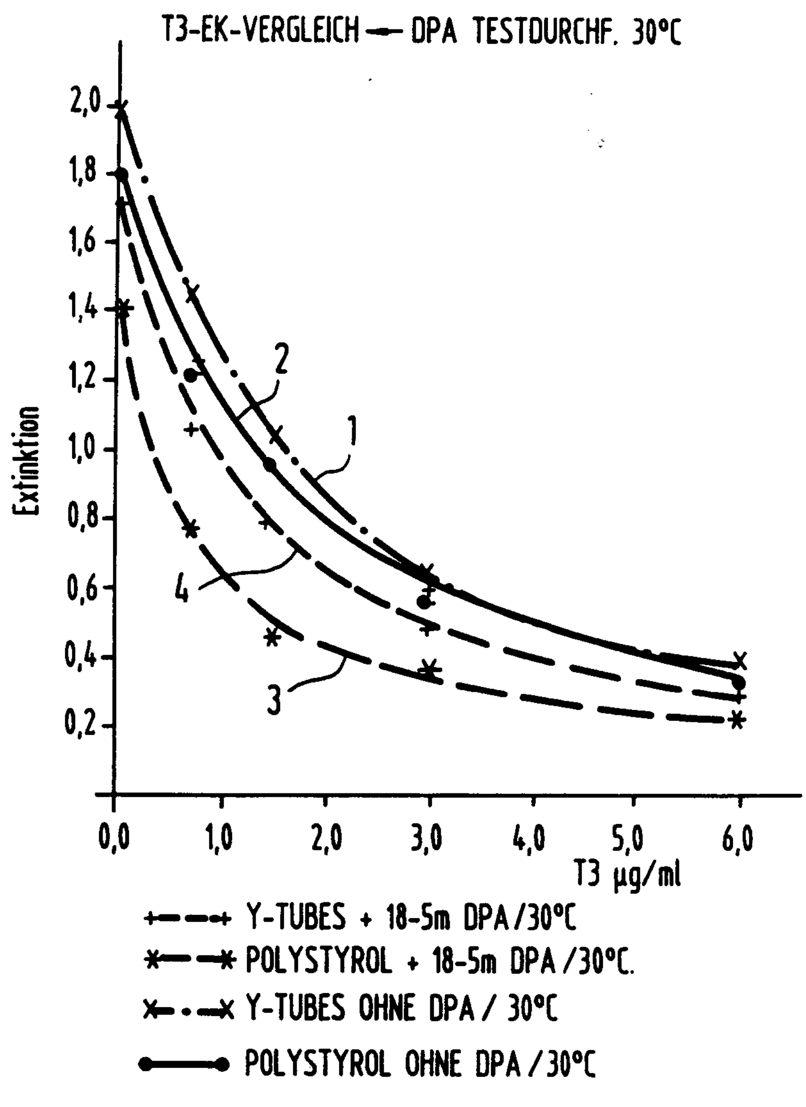
T3-EK-VERGLEICH — DPA TESTDURCHF. 30°C
Extinktion
2,0
1,8
1,6
1,4
1,2
1,0
0,8
0,6
0,4
0,2
1
2
3
4
0,0
1,0
2,0
3,0
4,0
5,0
6,0
T3 µg/ml
Y-TUBES + 18-5m DPA/30°C
POLYSTYROL + 18-5m DPA/30°C.
Y-TUBES OHNE DPA / 30°C
POLYSTYROL OHNE DPA/30°C